(19) Europäisches Patentamt
European Patent Office
Office européen des brevets

(11) Veröffentlichungsnummer: **0 148 117**
**B1**

(12) # EUROPÄISCHE PATENTSCHRIFT

(45) Veröffentlichungstag der Patentschrift: **04.01.89**

(51) Int. Cl.⁴: **C 07 D 303/36**, C 08 G 59/32

(21) Anmeldenummer: **84810576.3**

(22) Anmeldetag: **26.11.84**

(54) **Triglycidylverbindungen von Aminophenolen.**

(30) Priorität: **02.12.83 CH 6467/83**

(43) Veröffentlichungstag der Anmeldung: **10.07.85 Patentblatt 85/28**

(45) Bekanntmachung des Hinweises auf die Patenterteilung: **04.01.89 Patentblatt 89/1**

(84) Benannte Vertragsstaaten: **CH DE FR GB IT LI**

(56) Entgegenhaltungen:
**DE-B-1 268 152**
**DE-C-1 176 666**
**US-A-2 921 037**
**US-A-2 951 822**
**US-A-2 951 825**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Zahir, Sheik Abdul- Cader, Dr., Elsternstrasse 12, CH- 4104 Oberwil (CH)**

**EP 0 148 117 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue Triglycidylverbindungen von gegebenenfalls ringsubstituierten 4-Hydroxyphenyl-4-aminophenylalkanen, Epoxidharzgetische, enthaltend die neuen Triglycidylverbindungen und Tetraglycidylverbindungen bestimmter aromatischer Diamine, sowie die Verwendung der neuen Triglycidylverbindungen und deren Epoxidharzgenische in härtbaren Mischungen.

Tri- und tetrafunktionelle Glycidylverbindungen sind aufgrund des Eigenschaftsbildes, welches sie den aus diesen Epoxidharzen durch Härtung bzw. Vernetzung und Formgebung hergestellten Formstoffen verleihen, für viele technische Anwendungen interessant. So sind beispielsweise N,N-Diglycidyl-4-aminophenolglycidyläther (vgl. US-Patent 2 951 825) und N,N,N',N'-Tetraglycidyl-bis-(4-aminophenyl)-methan bekannte, im Handel erhältliche Verbindungen. Wie aus den im Journal of Applied Polymer Science, Vol. 26, 2363-2372 (1981) beschriebenen Untersuchungen hervorgeht, sind diese Glycidylverbindungen mit dem Mangel behaftet, feuchtigkeitsempfindlich zu sein, so dass entsprechende Vorkehrungen bei der Lagerung und Handhabung dieser Epoxidharze getroffen werden müssen. Ausserdem sind die beiden Glycidylverbindungen mit Härtungsmitteln für Epoxidharze sehr reaktiv [vgl. auch H. Lee und K. Neville: Handbook of Epoxy Resins (1967), 2-21], so dass B-Stufenharze nicht oder nur schlecht aus ihnen herstellbar sind.

Es wurde nun gefunden, dass Triglycidylverbindungen von gegebenenfalls ringsubstituierten 4-Hydroxyphenyl-4-aminophenyl-alkanen sowohl allein als auch im Gemisch mit Tetraglycidylverbindungen bestimmter aromatischer Diamine weniger feuchtigkeitsempfindlich sind und insbesondere zur Herstellung von B-Stufenharzen geeignet bzw. besser geeignet sind.

Gegenstand vorliegender Erfindung sind somit Triglycidylverbindungen der Formel I

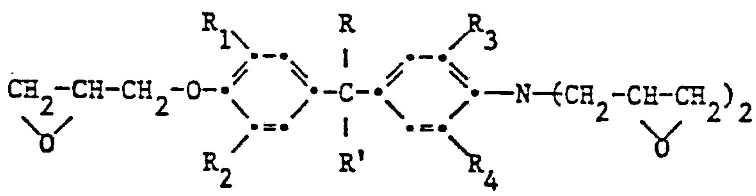 (I),

worin R und R' unabhängig voneinander je ein Wasserstoffatom oder Methyl, insbesondere Methyl, und $R_1$, $R_2$, $R_3$ und $R_4$ unabhängig voneinander je ein Wasserstoff-, Chlor-, Bromatom oder Alkyl mit bis zu 4 C-Atomen bedeuten.

Besonders interessant als Triglycidylverbindung der Formel I ist 2,2-(N,N-Diglycidyl-4-aminophenyl-4'-glycidyloxyphenyl)-propan.

Die vorliegende Erfindung betrifft auch Epoxidharzgemische, enthaltend (a) eine Triglycidylverbindung der Formel I und (b) eine Tetraglycidylverbindung der Formel II

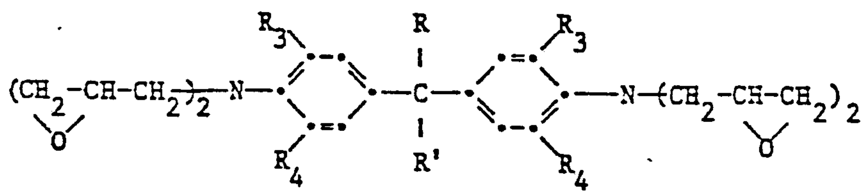 (II),

worin R, R', $R_3$ und $R_4$ die gleiche Bedeutung wie in Formel I haben und wobei im Epoxidharzgemisch das Verhältnis der Epoxidäquivalente von (a) zu (b) 10 : 1 bis 1 : 10 beträgt.

Ein bevorzugtes Epoxidharzgemisch enthält 2,2-(N,N-Diglycidyl-4-aminophenyl-4'-glycidyloxyphenyl)-propan als Verbindung der Formel I und N,N,N',N'-Tetraglycidyl-4,4'-diamino-diphenylmethan als Verbindung der Formel II.

Ferner beträgt im erfindungsgemässen Epoxidharzgemisch das Verhältnis der Epoxidäquivalente von (a) zu (b) vorzugsweise 5 : 1 bis 1 : 5, insbesondere 4 : 1 bis 1 : 4.

Die erfindungsgemässen Verbindungen der Formel I können hergestellt werden, indem man ein Aminophenol der Formel III

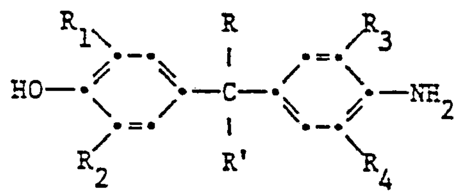

(III),

worin R, R', $R_1$, $R_2$, $R_3$ und $R_4$ die gleiche Bedeutung wie in Formel I haben, mittels eines Epihalogenhydrins oder Glycerindihalogenhydrins zu einer Verbindung der Formel I glycidyliert.

Die Glycidylierung von Hydroxyl- bzw. Aminogruppen aufweisenden organischen Verbindungen stellt eine bekannte Umsetzungsreaktion dar. Die Aminophenole der Formel III können beispielsweise mit Epichlorhydrin oder Glycerin-1,3-dichlorhydrin zu einem Tris-(chlorhydrin) der Formel IV

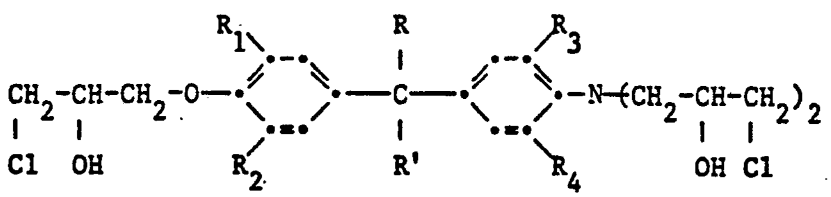

(IV)

umgesetzt werden, das dann dehydrochloriert wird, was vorzugsweise nach einer der folgenden Methoden durchgeführt wird:

A) In einem 2-Stufenverfahren wird zuerst das Aminophenol mit wenigstens 1,5, vorzugsweise mit 1,6 bis 5, Äquivalenten Epichlorhydrin oder Glycerin-1,3-dichlorhydrin in Gegenwart einer Lewissäure als Katalysator behandelt, wobei ein Tris-(chlorhydrin) der Formel IV entsteht. In der zweiten Stufe wird das Tris-(chlorhydrin) mit Alkali behandelt, wobei die Epoxidgruppen gebildet werden. Das Alkali ist normalerweise Na-hydroxid, doch können auch andere alkalische Substanzen, wie Ba-hydroxid oder K-carbonat für die Umwandlung der 1,2-Chlorhydringruppen in die 1,2-Epoxidgruppen verwendet werden.

B) Bei dem Einstufenverfahren wird das Aminophenol mit wenigstens 2,5, vorzugsweise 3 bis 8, Äquivalenten Epichlorhydrin in Gegenwart von Alkali, wie Na-Hydroxid, und eines Phasenumwandlungskatalysators, wie Tetramethylammoniumchlorid, eines tertiären Amins oder einer quaternären Ammoniumbase behandelt. Wenigstens ein Teil des Überschusses an Epichlorhydrin wirkt als Chlorwasserstoffakzeptor und begünstigt die Bildung der Glycidylgruppen, wobei dieser Teil des Epichlorhydrins in Glycerin-1,3-dichlorhydrin umgewandelt wird.

Die Umsetzung kann nach beiden Methoden in einem Lösungsmittel, beispielsweise Kohlenwasserstoffe, Äther oder Ketone, durchgeführt werden, doch wird bei dem Einstufenverfahren vorzugsweise ein Überschuss an Epichlorhydrin als Lösungsmittel verwendet. Die Umsetzung wird allgemein bei erhöhter Temperatur, etwa 40 - 100° C, durchgeführt.

Die Aminophenole der Formel III stellen bekannte Verbindungen dar.

Die Aminophenole der Formel III können beispielsweise nach dem im DE-Patent 1 176 666 oder den in der DE-AS-1 268 151 beschriebenen Verfahren hergestellt werden, indem man ein Bisphenol der Formel V

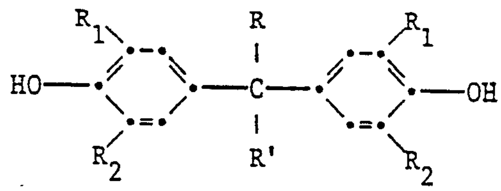 (V),

worin R, R', $R_1$ und $R_2$ die gleiche Bedeutung wie in Formel I haben, mit einem gegebenenfalls substituierten Anilin der Formel VI

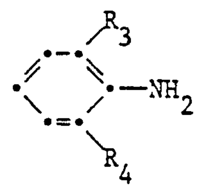 (VI),

worin $R_3$ und $R_4$ die gleiche Bedeutung wie in Formel I haben, gegebenenfalls in Gegenwart eines alkalischen, vorzugsweise sauren Katalysators umsetzt.

Geeignete Verbindungen der Formel V sind beispielsweise Bis-(4-hydroxphenyl)-methan (Bisphenol F), 2,2-Bis-(4-hydroxyphenyl)-propan (Bisphenol A), 2,2-Bis-(4-hydroxy-3,5-dibromphenyl)-propan (Tetrabronbisphenol A), 2,2-Bis-(3-methyl-4-hydroxyphenyl)-propan, 2,2-Bis-(3-chlormethyl-4-hydroxyphenyl)-propan, Bis-(3-tert.-butyl-4-hydroxyphenyl)-methan und 2,2-Bis-(3-Brom-4-hydroxyphenyl)-propan. Als substituierte Aniline seien beispielsweise 2,8-Diäthylanilin, 2-Isopropyl-6-methylanilin, 2,6-Dichloranilin, o-Chloranilin, o-Bromanilin und o-Toluidin genannt.

Die in den erfindungsgemässen Epoxidharzgemischen enthaltenen Tetraglycidylverbindungen der Formel II sind beispielsweise aus den US Patenten 2 921 037 und 2 951 822 bekannt und werden wie dort beschrieben durch Glycidylierung eines Diamins der Formel VII

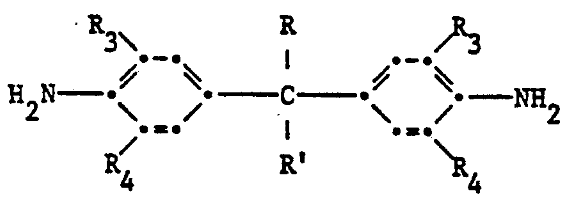 (VII),

worin R, R', $R_3$ und $R_4$ die gleiche Bedeutung wie in Formel II haben, mittels Epihalogenhydrin erhalten.

Die erfindungsgemässen Epoxidharzgemische werden vorzugsweise durch einfaches Mischen einer Triglycidylverbindung der Formel I mit einer Tetraglycidylverbindung der Formel II im angegebenen Epoxidäquivalentverhältnis hergestellt. Eine andere Möglichkeit zur Herstellung der erfindungsgemässen Epoxidharzgemische besteht in der Glycidylierung eines Gemisches aus einem Aminophenol der Formel III und einem Diamin der Formel VII im entsprechenden Äquivalentverhältnis.

Die erfindungsgemässen Triglycidylverbindungen und deren Gemische mit den Tetraglycidylverbindungen der Formel II können mit den üblichen Härtungsmitteln für Epoxidharze ausgehärtet werden. Ein weiterer Gegenstand vorliegender Erfindung sind somit härtbare Gemische, enthaltend eine Triglycidylverbindung der Formel I oder ein Gemisch aus einer Triglycidylverbindung der Formel I und einer Tetraglycidylverbindung der Formel II und ein Härtungsmittel für Epoxidharze.

Als Beispiele für Härtungsmittel seien die gebräuchlichen Härtungsmittel für Epoxidharze genannt, einschliesslich der aliphatischen, cycloaliphatischen, aromatischen und heterocyclischen Amine, wie Bis-(4-aminophenyl)-methan, Anilin-Formaldehyd-Harz, Bis-(4-aminophenyl)-sulfon, Propan-1,3-diamin, Hexamethylendiamin, Diäthylentriamin, Triäthylentetramin, 2,2,4-Trimethylhexan-1,6-diamin, m-Xylylendiamin, Bis-(4-aminocyclohexyl)-methan, 2,2-Bis-(4-aminocyclohexyl)-propan und 3-Aminomethyl-3,5,5-trimethylcyclohexylamin (Isophorondiamin), Polyaminoamide, wie beispielsweise solche aus aliphatischen Polyaminen und dimerisierten oder trimerisierten Fettsäuren, Polyphenole, wie Resorcin, Hydrochinon, 2,2-Bis-(4-hydroxyphenyl)-propan und Phenol-Aldehyd-Harze, Polythiol, wie die im Handel unter der Bezeichnung "Thiokole" erhältlichen Polythiole, Polycarbonsäuren und ihre Anhydride, wie zum Beispiel Phthalsäureanhydrid, Tetrahydrophthalsäureanhydrid, Hexahydrophthalsäureanhydrid, Hexachlorendomethylentetrahydrophthalsäureanhydrid, Pyromellitsäureanhydrid, Benzophenon-3,3',4,4'-tetracarbonsäuredianhydrid, die Säuren der zuvorgenannten Anhydride sowie auch Isophthalsäure und Terphthalsäure. Es können auch katalytisch wirkende Härtungsmittel verwendet werden, die beispielsweise tertiäre Amine [zum Beispiel 2,4,6-Tris-(dimethylaminoäthyl)-phenol], Imidazole und andere Mannichbasen; Alkalimetallalkoxide von Alkoholen (zum Beispiel Na-Alkoholat von 2,4-Dihydroxy-3-hydroxymethylpentan), Zinnsalze von Alkansäuren (zum Beispiel Zinnoctanoat), Friedel-Crafts-Katalysatoren, wie Bortrifluorid und Bortrichlorid und ihre Komplexe und Chelate, die durch Umsetzung von Bortrifluorid mit zum Beispiel 1,3-Diketonen erhalten werden.

Mit den Härtungsmitteln können auch die geeigneten Härtungsbeschleuniger eingesetzt werden. Bei Verwendung von Poly-(aminoamiden), Polythiole oder Polycarbonsäureanhydriden können tertiäre Amine oder deren Salze, quaternäre Ammoniumverbindungen oder Alkalimetallalkoxide als Beschleuniger dienen.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Wenn das Härtungsmittel ein Amin ist, werden normalerweise 0,75 bis 1,25 Äquivalente Aminwasserstoff pro 1 Epoxidäquivalent eingesetzt. Wenn Polycarbonsäuren oder ihre Anhydride eingesetzt werden, verwendet man gewöhnlich 0,4 bis 1,1 Äquivalente Caroxylgruppe bzw. Anhydridgruppe per 1 Äquivalent Epoxidgruppe. Bei der Verwendung von Polyphenolen als Härtungsmittel setzt man zweckmässig 0,75 bis 1,25 phenolische Hydroxylgruppen per 1 Epoxidäquivalent ein.

Katalytisch wirkende Härtungsmittel werden allgemein in Mengen von 1 bis 40 Gewichtsteilen pro 100 Gewichtsteile Epoxidharz eingesetzt.

Je nach Natur des verwendeten Härtungsmittels kann die Härtung bei Raumtemperatur oder bei höheren Temperaturen vorgenommen werden. Die Härtung kann gewünschtenfalls auch in zwei Stufen vorgenommen werden, indem man zum Beispiel den Härtungsvorgang unterbricht oder, falls man ein Härtungsmittel für höhere Temperaturen einsetzt, die härtbare Mischung bei tieferen Temperaturen teilweise härten lässt. Die dabei erhaltenen Produkte sind noch schmelzbare und lösliche Präkondensate (sogenannte "B-Stufenharze") und eignen sich z. B. für Pressmassen, Sinterpulver oder Prepregs.

Die erfindungsgemässen härtbaren Mischungen können ferner noch Plastifizierungsmittel, wie Dibutylphthalat, Dioctylphthalat oder Trikresylphosphat, oder Additive erhalten, wie Streckmittel, Füllstoffe, Verstärkungsmittel, Färbemittel, Fliessmittel und Formtrennmittel. Geeignete Streckmittel, Füllstoffe und Verstärkungsmittel sind beispielsweise Asbest, Asphalt, Bitumen, Glasfasern, Textilfasern, Kohlenstoff- oder Borfasern, Glimmer, Tonerde, Gips, Titandioxid, Kreide, Quarzmehl, Cellulose, Kaolin, gemahlener Dolomit, Wollastonit, Kieselerde mit grosser spezifischer Oberfläche (erhältlich unter dem Handelsnamen "Aerosil"), mit langkettigen Aminen modifizierte Tonerde (erhältlich unter dem Handelsnamen "Bentone"), gepulvertes Polyvinylchlorid, Polyolefin oder Aminoplaste, Metallpulver, wie Aluminium- oder Eisenpulver. Flammschutzmittel, wie Antimontrioxid, können ebenfalls den härtbaren Mischungen zugegeben werden.

Die erfindungsgemässen härtbaren Massen lassen sich z. B. als Laminier-, Tränk- und Giessharze, Pulverbeschichtungen, Formmassen, Kitte und Dichtungsmassen, Einbettungs- und Isoliermassen für die Elektrotechnik und insbesondere als Klebstoffe oder Matrixharze zur Herstellung von faserverstärkten Kunststoffen verwenden.

**Beispiel 1:**

Herstellung von

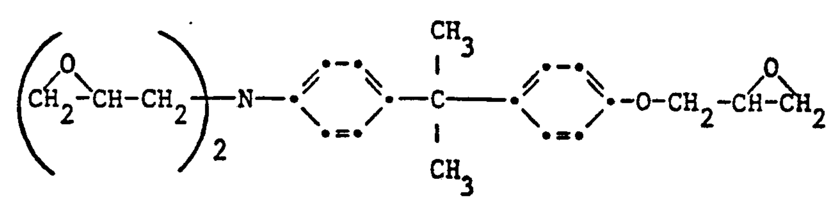

In einem mit Rührer, Thermometer, Dean-Stark-Abscheider, Rückflusskühler und Tropftrichter versehenen Reaktionsgefäss werden 272,4 g (1,2 Mol) 2,2-(4-Aminophenyl-4'-hydroxyphenyl)-propan, hergestellt gemäss Beispiel 2 der DE-AS-1 268 152, und 1170 g (12,62 Mol) Epichlorhydrin vorgelegt, gemischt und die Aufschlämmung unter Rühren auf 95°C erhitzt. Es wird eine klare Lösung erhalten, die eine Stunde bei 95°C weitergerührt wird. Dann werden 5,81 g einer 50-%-igen wässrigen Tetramethylammoniumchloridlösung zugegeben, und die Reaktionslösung wird während 2 Stunden bei 90 bis 95°C gerührt. Dann wird sie auf 20°C abgekühlt und durch den Tropftrichter gibt man 187,2 g einer 50-%-igen wässrigen NaOH-Lösung zu, wobei die Temperatur des Reaktionsgemisches auf 20°C gehalten wird. Dann erhitzt man die Reaktionslösung auf 50°C erhitzt und unter zunehmendem verminderten Druck (160 → 53 mbar) werden während zwei Stunden Wasser und Epichlorhydrin azeotrop aus der Reaktionslösung entfermt. Dann werden 144,0 g der 50-%-igen wässrigen NaOH-Lösung zugegeben und das Wasser und Epichlorhydrid während 2 Stunden bei 50°C/160 mbar azeotrop aus der Reaktionslösung entfernt. Nach Abkühlen auf Raumtemperatur wird die organische Phase in Methylenchlorid aufgenommen, mehrmals mit Wasser gewaschen und über wasserfreiem Na-sulfat

getrocknet. Nach Entfernen der Lösungsmittelreste und des nichtreagierten Epichlorhydrins mittels Destillation bei 50° C/20 mbar werden 382 g (81 % der Theorie) eines starkbraunen Harzes mit einem Epoxidgehalt von 6,19 Äquivalenten/kg und einer Viskosität von 5 500 mPa·$s^{-1}$ bei 50° C erhalten.

**Anwendungsbeispiele**

Die in der folgenden Tabelle angegebenen härtbaren Mischungen aus 2,2-(N,N-Diglycidyl-4-aminophenyl-4'-glycidyloxyphenyl)-propan (A) gemäss Beispiel 1, N,N,N',N'-Tetraglycidyl-4,4-diaminodiphenylmethan (B) und 4,4'-Diaminodiphenylmethan (C) als Härtungsmittel werden zu Formkörpern verarbeitet, indem aus den härtbaren Mischungen bei 120° C klare Schmelzen hergestellt werden, die, bevor sie in auf 150° C vorgewärmte Aluminiumformen gegossen werden, unter Vakuum bei 20 mbar von eingeschlossenen Luftblasen befreit werden und anschliessend 2 Stunden bei 120° C, 2 Stunden bei 150° C und 2 Stunden bei 180° C gehärtet werden. Aus den erhaltenen Formkörpern werden entsprechend der angegebenen Norm Prüfkörper geschnitten, deren mechanische Eigenschaften in der folgenden Tabelle angegeben werden.

**Tabelle**

| | | | | |
|---|---|---|---|---|
| Härtbare Mischung aus: | | | | |
| | A | 847,0 g (5 Äq.) | 508,0 g (3 Äq.) | 423,7 g (2.5 Äq.) |
| | B | - | 250,0 g (2 Äq.) | 312,5 g (2,5 Äq.) |
| | C | 247,5 g (5 Äq.) | 247,5 g (5 Äq.) | 247,5 g (5 Äq.) |
| Gelierzeit (Sekunden) | | | | |
| bei: | 120° C | 750 | 890 | 830 |
| | 150° C | 275 | 325 | 320 |
| | 180° C | 105 | 115 | 120 |
| Viskosität bei 50° C (mPa·$s^{-1}$) | | | | |
| | 0 Min. | 4641 | 4641 | 5355 |
| | 60 Min. | 10710 | 8211 | 9639 |
| Formbeständigkeit in der Wärme nach ISO*) 75 (° C) | | 159 | 186 | |
| Biegefestigkeit nach ISO 178 (N/mm$^2$) | | 137,7 | 133,1 | |
| E-Modul nach ISO 178 (N/mm$^2$) | | 3772 | 3865 | |

| | | | |
|---|---|---|---|
| Härtbare Mischung aus: | | | |
| | A | 339,0 g (2 Äq.) | 169,0 g (1 Äq.) |
| | B | 375,0 g (3 Äq.) | 500,0 g (4 Äq.) |
| | C | 247,5 g (5 Äq.) | 247,5 g (5 Äq.) |
| Gelierzeit (Sekunden) | | | |
| bei: | 120° C | 1035 | 900 |
| | 150° C | 330 | 315 |
| | 180° C | 110 | 105 |
| Viskosität bei 50° C (mPa·$s^{-1}$) | | | |
| | 0 Min. | 4284 | 6247 |
| | 60 Min. | 6961 | 9961 |
| Formbeständigkeit in der Wärme nach ISO*) 75 (° C) | | 194 | 210 |
| Biegefestigkeit nach ISO 178 (N/mm$^2$) | | 146,3 | 136,1 |
| E-Modul nach ISO 178 (N/mm$^2$) | | 3840 | 3576 |

*) ISO = International Standard Organization

**Patentansprüche**

1. Triglycidylverbindungen der Formel I

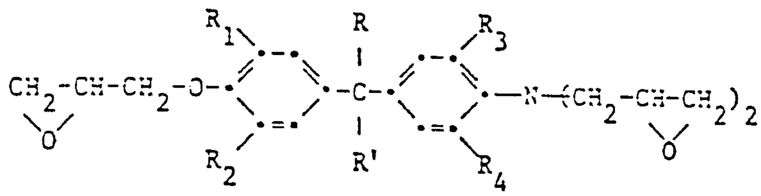

(I),

worin R und R' unabhängig voneinander je ein Wasserstoffatom oder Methyl und $R_1$, $R_2$, $R_3$ und $R_4$ unabhängig voneinander je ein Wasserstoff-, Chlor-, Bromatom oder Alkyl mit bis zu 4 C-Atomen bedeuten.

2. 2,2-(N,N-Diglycidyl-4-aminophenyl-4'-glycidyloxyphenyl)-propan als Triglycidylverbindung der Formel I gemäss Anspruch 1.

3. Epoxidharzgemische, enthaltend (a) eine Triglycidylverbindung der Formel I gemäss Anspruch 1 und (b) eine Tetraglycidylverbindung der Formel II

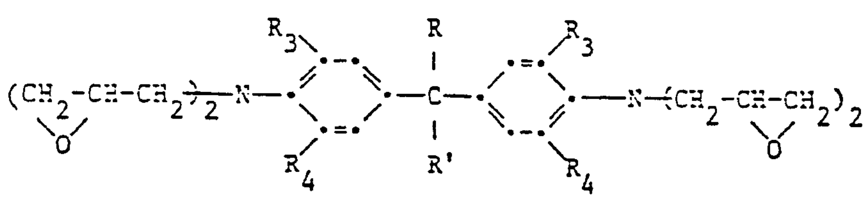

(II),

worin R, R', $R_3$ und $R_4$ die gleiche Bedeutung wie in Formel I gemäss Anspurch 1 haben und wobei im Epoxidharzgemisch das Verhältnis der Epoxidäquivalente von (a) zu (b) 10 : 1 bis 1 : 10 beträgt.

4. Epoxidharzgemische genäss Anspruch 3, enthaltend 2,2-(N,N-Diglycidyl-4-aminophenyl-4'-glycidyloxyphenyl)-propan als Verbindung der Formel I und N,N,N',N'-Tetraglycidyl-4,4'-diaminodiphenylmethan als Verbindung der Formel II.

5. Epoxidharzgemische gemäss Anspruch 3, worin das Verhältnis der Epoxidäquivalente von (a) zu (b) 5 : 1 bis 1 : 5 beträgt.

6. Härtbare Mischungen, enthaltend eine Triglycidylverbindung der Formel I gemäss Anspruch 1 oder ein Gemisch aus einer Triglycidylverbindung der Formel I und einer Tetraglycidylverbindung der Formel II gemäss Anspruch 3 und ein Härtungsmittel für Epoxidharze.

**Revendications**

1. Composés triglycidyliques répondant à la formule I:

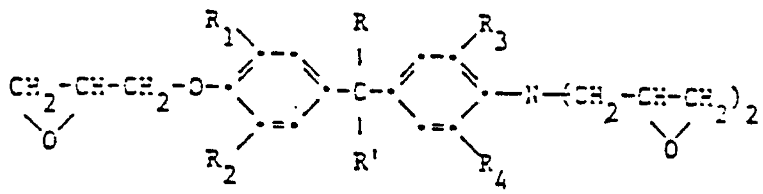

(I)

dans laquelle R et R' représentent chacun, indépendamment l'un de l'autre, un atome d'hydrogène ou un radical méthyle, et $R_1$, $R_2$, $R_3$ et $R_4$ représentent chacun, indépendamment les uns des autres, un atome d'hydrogène, de chlore ou de brome ou un radical alkyle contenant au plus 4 atomes de carbone.

2. Composé triglycidylique de formule I selon la revendication 1, en l'espèce le (diglycidylamino-4 phényl)-2 (glycidyloxy-4 phényl)-2 propane.

3. Mélanges à base de résines époxydiques qui contiennent (a) un composé triglycidylique de formule I selon la revendication 1 et (b) un composé tétraglycidylique répondant à la formule II:

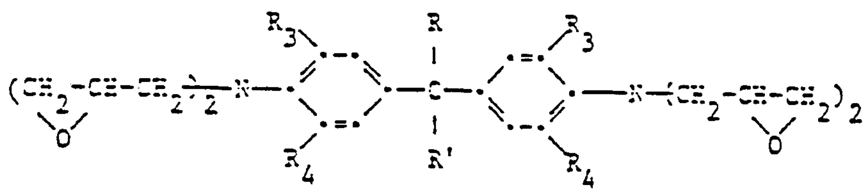

(II)

dans laquelle R, R', $R_3$ et $R_4$ ont chacun les mêmes significations que dans la formule I représentée à la revendication 1, le rapport entre le nombre d'équivalents d'époxy de (a) et celui de (b) étant compris, dans le mélange de résines époxydiques entre 1 : 10 et 1 : 10.

4. Mélanges à base de résines époxydiques selon la revendication 3 qui contiennent, comme composé de formule I, du (diglycidylamino-4 phényl)-2 (glycidyloxy-4 phényl)-2 propane et, comme composé de formule II, du N,N,N',N'-tétraglycidyl-diamino-4,4' diphénylméthane.

5. Mélanges à base de résines époxydiques selon la revendication 3 dans lesquels le rapport entre le nombre des équivalents d'époxy de (a) et celui de (b) est compris entre 5 : 1 et 1 : 5.

6. Mélanges durcissables qui contiennent un composé triglycidylique de formule I selon la revendication 1, ou un mélange d'un composé triglycidylique de formule I et d'un composé tétraglycidylique de formule II selon la revendication 3, ainsi qu'un durcisseur de résines époxydiques.

**Claims**

1. A triglycidyl compound of the formula I

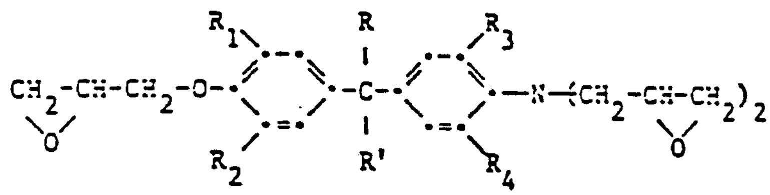

(I)

wherein each of R and R' independently is a hydrogen atom or methyl, and each of $R_1$, $R_2$, $R_3$ and $R_4$ independently is a hydrogen, chlorine or bromine atom or alkyl of up to 4 carbon atoms.

2. 2,2-(N,N-Diglycidyl-4-aminophenyl-4'-glycidyloxyphenyl)-propane as triglycidyl compound of the formula I according to claim 1.

3. An epoxy resin mixture containing (a) a triglycidyl compound of the formula I according to claim 1 and (b) a tetraglycidyl compound of the formula II

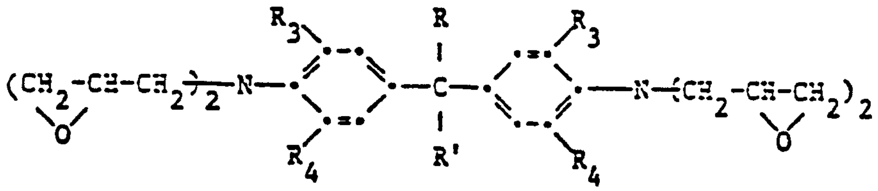

(II)

wherein R, R', $R_3$ and $R_4$ are as defined for formula I according to claim 1, the ratio of the epoxy equivalents of (a) to (b) in the epoxy resin mixture being 10 : 1 to 1 : 10.

4. An epoxy resin mixture according to claim 3, containing 2,2-(N,N-diglycidyl-4-aminophenyl-4'-glycidyloxyphenyl)-propane as compound of the formula I and N,N,N',N'-tetraglycidyl-4,4'-diaminodiphenylmethane as compound of the formula II.

5. An epoxy resin mixture according to claim 3, wherein the ratio of epoxy equivalents of (a) to (b) is 5 : 1 to 1: 5.

6. A curable mixture containing a triglycidyl compound of the formula I according to claim 1 or a mixture of a triglycidyl compound of the formula I and a tetraglycidyl compound of the formula II according to claim 3, and a curing agent for epoxy resins.